## Europäisches Patentamt

(19) **European Patent Office**

**Office européen des brevets**

(11) Publication number: **0 209 911**
**B1**

---

(12) # EUROPEAN PATENT SPECIFICATION

(45) Date of publication of the patent specification:
06.12.89

(21) Application number: **86110274.7**

(22) Date of filing: **25.07.86**

(51) Int. Cl.⁴: **C07D 301/08**, C07D 303/48, B01J 32/00, B01J 21/08, B01J 35/10

---

(54) Process and catalyst for oxidizing fluorinated olefins.

---

(30) Priority: **26.07.85  IT 2173185**

(43) Date of publication of application:
**28.01.87 Bulletin 87/5**

(45) Publication of the grant of the patent:
**06.12.89 Bulletin 89/49**

(84) Designated Contracting States:
**BE DE FR GB NL SE**

(56) References cited:
GB-A- 2 099 327
US-A- 3 775 438

PATENT ABSTRACTS OF JAPAN, vol. 1, no. 97, 30th August 1977, page 2020 C 77; & JP-A-52 53 805 (ASAHI GLASS K.K.) 30-04-1977
PATENT ABSTRACTS OF JAPAN, vol. 1, no. 97, 30th August 1977, page 2020 C 77; & JP-A-52 53 806 (ASAHI GLASS K.K.) 30-04-1977

(73) Proprietor: **MONTEDIPE S.p.A., 31, Foro Buonaparte, I-20121 Milan(IT)**

(72) Inventor: **Castellan, Arsenio, 11, via Ruzzante, I-28100 Novara(IT)**
Inventor: **Gregorio, Guglielmo, 7/2, via Fabriano, I-20100 Milan(IT)**
Inventor: **Padovan, Mario, 1, via Mirabello, I-20125 Milan(IT)**

(74) Representative: **Barz, Peter, Dr. et al, Patentanwälte Dipl.-Ing. G. Dannenberg Dr. P. Weinhold, Dr. D. Gudel Dipl.-Ing. S. Schubert, Dr. P. Barz Siegfriedstrasse 8, D-8000 München 40(DE)**

---

ACTORUM AG

## Description

This invention relates to a process and a catalyst for oxidizing fluorinated olefins to the corresponding epoxides and, particularly, the present invention relates to a process and a catalyst for oxidizing tetrafluoroethylene and hexafluoropropene.

Processes for oxidizing fluorinated olefins, for example, tetrafluoroethylene and hexafluoropropene to the corresponding epoxides, are known in the art and have, for instance, been described in US patents 3 775 438, 3 775 439 and 3 775 440.

According to these patents, hexafluoropropene and tetrafluoroethylene are oxidized with oxygen, either pure or mixed with inert gases, in the presence of a catalyst essentially consisting of silica. The catalyst, which has an SiO$_2$ content ranging from 60 to 95% by weight, is used in different forms, for instance as a gel, as ground glass, as macroporous beads or also as sand.

Although these processes allow sufficiently high yields and conversions to epoxides, they have certain limitations which render them unsuitable for utilisation on a commercial scale. One such limitation is the catalyst life; in fact, the catalyst becomes deactivated unless it is continuously treated with water or steam.

Another limitation is due to the fact that, under certain conditions, the oxidation reaction requires a pressure that is higher than atmospheric pressure or the use of high temperatures; in both cases there are considerable drawbacks: In the former case, the hazard of working under pressure, in the latter case, the risk of employing a temperature close to the decomposition temperature of epoxides, resulting in a reduction of the yield of the final product.

Consequently, other processes for preparing hexafluoropropene oxide in particular have been proposed and are, for example, described in JP-A-77-53804, 77-53805 and 77-53806. As in the other cases, oxidation occurs by direct contact between fluoroolefin and catalyst but the catalyst is composed of a product based on silica which carries promoters selected from transition metals such as copper, chromium, manganese, iron, zinc, palladium and cerium.

There are, however, still shortcomings limiting the described processes, mainly as regards conversion and reaction yield. The corresponding values are very low, also owing to reaction temperatures which are too high, ranging from 250 to 300°C.

Thus, it is an object of the present invention to provide a process for oxidizing fluorinated olefins which avoids the drawbacks mentioned above.

More particularly, it is an object of the present invention to provide a process for oxidizing fluorinated olefins C$_2$-C$_6$ and, in particular, tetrafluoroethylene and hexafluoropropene with high conversions and yields under reaction conditions which are consistent with the stability of the product.

Another object of the present invention is to provide a catalyst suitable for the oxidation of fluoroolefins to epoxides.

It has now surprisingly been found that these and still other objects can be achieved by conducting the direct reaction between fluorinated olefin and oxygen in the presence of an oxidizing catalyst consisting of a crystalline form of porous silicon dioxide having high purity and carrying oxides of metals of the first transition series or of lanthanides or mixtures thereof.

The term "metals of the first transition series" refers to those metals in the Periodic Table between scandium and zinc. Preferred metals are copper, cobalt, cerium and chromium.

The process according to the present invention can be conducted either continuously or discontinuously. According to a preferred embodiment, the oxidation reaction is conducted continuously by conveying the fluorinated olefin and the oxygen over a fixed or fluid catalytic bed and by keeping the reactants in contact for a time of from 100 to 1000 seconds.

Oxygen can be fed in the form of air or of another gas mixture containing at least 20% by volume of oxygen. The diluting gas is inert and can, for example, be helium or carbon dioxide. Generally, it is preferred to use pure oxygen at a concentration, in the reaction mixture, of from 1 to 60% by volume.

Prior to the oxidation reaction, the catalyst is subjected to an activation step which can be accomplished by contacting it either with the reactants themselves or with fluoro-derivatives selected from dichlorodifluoromethane, tetrafluoromethane, hexafluoroethane, tetrafluoroethylene, hexafluoropropene, gaseous hydrofluoric acid or acid fluorides such as trifluoroacetylfluoride or carbonyl difluoride, either pure or diluted in an inert gas such as nitrogen, at temperatures of from 0 to 300°C, depending on the activator type.

The oxidation reaction is conducted at atmospheric pressure or slightly above it to overcome the pressure drop due to the catalytic bed and at temperatures up to 250°C. If the produced epoxide is that of tetrafluoroethylene, the reaction temperature is lower, preferably below 50°C. Conversely, if the produced epoxide is that of a fluoroolefin having a number of carbon atoms from 3 to 6, the temperature preferably ranges from 50 to 250°C.

The expoxide thus obtained can be recovered from the reaction mixture according to any known method, such as distillation or washing.

The catalyst according to the present invention, which comprises a carrier and metal oxides, exhibits excellent catalytic properties in oxidation reactions, in particular, in the oxidation of fluoroolefins to epoxides.

The carrier is a crystalline form of porous silicon dioxide having high purity and an aluminium content of below 0.1% by weight, characterized by an X-ray diffraction pattern (Cu K$^-\alpha$) with the following main reflection angles: Z$\vartheta$ = 7.94°; 8.85°; 23.08°; 23.94°.

The structure of the pure crystalline phase is porous with regular pores. Such characteristic is evidenced by the specific surface area values determined by absorption of nitrogen, said surface area being attributable for over 50% to pores with an average diameter below 1 nm. The total surface area ranges from 300 to 600 m$^2$/g.

The morphology of the crystallites varies. The individual crystallites appear in the octahedral or prismatic form with geminate prisms having an average size about 300 nm. They can, in any case, easily be distinguised from any amorphous materials by means of electron scanning microscopy.

The carrier can be prepared according to known methods, such as, for example, those described in Nature, Vol. 271, page 512 (1978) or is, for example, commercially available under the trademark SILICATE®.

The promoters, which consist of oxides of metals of the first transition series, i.e., those elements in the Periodic Table between scandium and zinc, or of lanthanides or of mixtures thereof, are added to the carrier by impregnating the same either with the oxide or with a salt capable of being decomposed by calcination to result in the corresponding oxide.

The promoters can be carried in amounts even higher than 40% by weight and, according to a preferred formulation of the catalyst of the present invention, amounts of from 1 to 30% by weight are utilized.

Any metal oxide of the above-cited group is capable of promoting the oxidation reaction of fluorinated olefins, however, preferred metal oxides are those of copper, cobalt, cerium and chromium.

The carrier impregnated with the transition metal salt is calcined in an oven at temperatures ranging from 300 to 800°C and is then subjected to an activating treatment as described above.

The following examples serve to further illustrate the present invention without, however, limiting it.

Example 1

90 of commercial SILICATE® (S-115 Union Carbide) were impregnated with 39 ml of an aqueous solution containing 18.6 g of $Cu(NO_3)_2.3H_2O$. After impregnation the catalyst was allowed to digest for 4 hours at room temperature, whereafter it was dried at 120°C for 16 hours.

It was calcined at 500°C for 4 hours in air, whereupon 4.5 g of an inert binder (bentonite) were added and the whole was kneaded with 45 ml of distilled water. It was dried at 120°C for 16 hours and calcined at 540°C for 2 hours in air. Finally, the catalyst was ground and subjected to screening. The 32-80 mesh fraction was collected and activated at 100°C for 5 hours in a nitrogen stream containing 0.1% of hydrofluoric acid.

On the spectrophotometric analysis (X-ray) the catalyst exhibited the following reflexes attributable to copper oxide:
$2\vartheta(Cu\ K-\alpha) = 38.75°; 36.60°; 48.97°$
besides the following, much more intense reflexes of the crystalline silica used as carrier:
$2\vartheta= 7.94°; 8.85°; 23.08°; 23.94°$.

30 g of the catalyst thus prepared were introduced into a cylindrical 50-cc reactor made of Incoloy and externally heated. The reactor was heated to 170°C and a gaseous mixture of hexafluoropropene and oxygen at a ratio of 90:70, namely 90 cc/h of hexafluoropropene and 70 cc/h of oxygen was introduced.

After about 1 hour the temperature was adjusted to 160°C (maximum temperature within the catalytic bed). The outflowing gases were subjected to gas-chromatographic analysis and their composition was found to be the following: $O_2 = 14.1\%$; $CO_2 + COF_2 = 21.8\%$; $C_3F_6O = 21.1\%$; $C_3F_6 = 30.6\%$.

This corresponded to a conversion of perfluoropropene of 41.3% and a selectivity to the epoxide of 75.6%.

After a 27-hour run the gas composition had slightly changed as follows: $O_2 = 17\%$; $CO_2+COF_2 = 23.7\%$; $C_3F_6O = 21.8\%$; $C_3F_6 = 35.2\%$, which corresponded to a conversion of hexafluoropropene of 45.5% with a selectivity to the epoxide of 74.5%.

Example 2

A mixture consisting of 180 cm³/h of $C_3F_6$ and 140 cm³/h of $O_2$ was made to flow over 30 g of catalyst which had been prepared according to the procedure of example 1 but had a mean diameter of 2 mm and had been activated for 3 hours with a nitrogen flow containing 0.5% of hydrofluoric acid, while the temperature was maintained at an average of 140°C, and not exceeding 147°C.

After 4 hours the conversion and the selectivity were stabilized with practically constant values, i.e., a conversion of about 30% and a selectivity for $C_3F_6O$ of higher than 77% from the 5th to the 50th hour of run.

A typical analysis of the outflowing gases after 43 h and 30 minutes was the following: $O_2 = 20.4\%$; $CO_2 + COF_2 = 11.7\%$; $C_3F_6O = 17.5\%$; $C_3F = 50.1\%$, corresponding to a conversion of 29.7% and to a selectivity of 82.6%.

After an 80-hour run the catalyst was cooled and discharged. The sample, subjected to X-ray analysis, still revealed the reflexes of the starting SILICALITE with an intensity comparable to that of the starting sample.

Example 3

3.5 g of a catalyst prepared as in example 1 (size: 32-80 mesh) were introduced into a 10-cc microreactor made of Incology. The reactor was heated to 200°C and a mixture consisting of 90 cc/h of hexafluoropropene and 70 cc/h of oxygen was made to flow on the catalytic bed thus prepared. After 1 hour the heating was adjusted in such a manner that, at the warmest point of the catalytic bed, the temperature was 200°C.

A practically constant conversion of the gas was observed and, after a 14-hour run, the analysis was as follows:
$O_2 = 25.16\%$; $CO_2 + COF_2 = 9.8\%$; $C_3F_6O = 9.7\%$; $C_3F_6 = 53.7\%$, which corresponded to a conversion of 19.15% and to a selectivity of 75.9%.

Example 4

A catalyst was prepared under the same conditions as described in example 1, except for the amount of $Cu(NO_3)_2.3H_2O$ which, in the present example, was 3.7 g. The final activation was carried out for 1 hour at 100°C in a nitrogen stream containing 0.5% of hydrofluoric acid.

3.5 g of the catalyst thus prepared having a size of 32-80 mesh were introduced into a 10-cc microreactor made of Incoloy. The reactor was heated to 200°C and a mixture of 90 cc/h of hexafluoropropene and 70 cc/h of $O_2$ was introduced.

The composition of the outflowing gases stabilized after 2 hours and after 7 hours was as follows: $O_2$ = 8%; $CO_2$ + $COF_2$ = 16.5%; $C_3F_6O$ = 13.5%; $C_3F_6$ = 52.0%, which corresponded to a conversion of 27% and to a selectivity of 69%.

Example 5

10 g of a catalyst having a size of 32-80 mesh and consisting of cobalt oxide on silicalite, which had been prepared as in example 1 using, however, cobalt nitrate instead of copper nitrate with the same amount of metal in gram atoms, were introduced into a 20-cc microreactor and heated to 230°C.

A mixture consisting of 90 cc/h of $C_3F_6$, 70 cc/h of $O_2$ and 30 cc/h of $N_2$ was introduced. The regular operating conditions were reached after a few hours and the temperature was stabilized at 180°C with a conversion of about 30%.

After a 6-hour run the conversion was stable and the outflowing mixture had the following composition:
$O_2$ + $N_2$ = 48.3%; $CO_2$ + $COF_2$ = 4.2%; $C_3F_6O$ = 12.4%; $C_3F_6$ = 35.5%, which corresponded to a conversion of 31% and to a selectivity to the epoxide of 74.7%.

Example 6

3.5 g of a catalyst having a size of 32-80 mesh and consisting of cerium oxide on silicalite, prepared as in example 1 using, however, cerium nitrate instead of copper nitrate, with the same amount of metal in gram atoms, were introduced into a 10-cc microreactor. This was heated to 230°C and a mixture of 90 cc/h of $C_3F_6$ + 70 cc/h of $O_2$ + 30 cc/h of $N_2$ was introduced over a period of half an hour, whereafter the temperature was gradually lowered. Regular operating conditions were reached at 195°C and, at this temperature, a relatively stable conversion was observed. After 4 hours an analysis of the outflowing gases revealed the following compositions:
$O_2$ + $N_2$ = 38%; $CO_2$ + $COF_2$ = 14.8%; $C_3F_6O$ = 8.6%; $C_3F_6$ = 36.6%. The conversion was 27.9% and the selectivity 61.2%.

Example 7 (Comparative Test)

90 g of a commercial, activated carbon designated as KAS/S HYDRO-LINE were ground and screened and the 32-80 mesh fraction was collected.

It was impregnated by means of the double-impregnation technique with an aqueous solution containing 9 g of $Cu(CH_3COO)_2.H_2O$. Then, it was dried at 100°C in air for 16 hours and was calcined in nitrogen at 300°C for 4 hours and, then, in air at 200°C for 4 hours.

3 g of the catalyst thus prepared were introduced into a 10-cc microreactor. This was heated to 100°C and a mixture of 90 cc/h of hexafluoropropene and 70 cc/h of oxygen was introduced. During the test, the temperature was increased from 100 to 200°C. The conversion of hexafluoropropene was practically complete; the outflowing gases revealed no traces of hexafluoropropene epoxide but of other oxidation and pyrolysis products such as $COF_2$, $CO_2$, $CF_4$, $CF_3COF$, $C_2F_4$, cyclic $C_3F_6$ and other high-boiling products.

Example 8 (Comparative Test)

50 ml of distilled water were added to 50 g of commercial silical sol (Ketjensol). Then, the mixture was acidified with dilute $HNO_3$ up to a pH = 6, and 4 g of $Cu(NO_3)_2.3H_2O$ were added.

Precipitation was carried out with an aqueous solution of KOH at 10%. The mixture was filtered, repeatedly washed and dried in air at 120°C/16 h, then calcined in air at 500°C for 4 hours, whereafter the catalyst was subjected to grinding and screening and the 32-80 mesh fraction was collected.

3.5 g of the catalyst thus prepared were introduced into a 10-cc microreactor. A mixture of 90 cc/h of hexafluoropropene and 70 cc/h of oxygen was made to pass over this catalytic bed while the reactor was heated to 300°C. At 300°C the conversion began and, after 1 hour, the temperature was decreased to 160°C, whereafter it was maintained constant.

The outflowing gases were almost exclusively composed of the unaltered reactants. It was then heated to 210°C and, under these conditions, it was possible to observe the following composition of the reacted mixture: $CO_2$ + $COF_2$ = 10.2%; $O_2$ = 36.5%; $C_3F_6$ = 51.8%, with traces of epoxide. The conversion was 15% but the selectivity to the epoxide was lower than 1%.

Example 9 (Comparative Test)

A catalyst consisting of copper supported on a zeolite-containing aluminium was prepared by ion exchange with copper nitrate on synthetic Fujasite (LZY-52 available from Union Carbide). The resulting product was calcined at 540°C and subjected to a nitrogen stream containing 0.1% of hydrofluoric acid at 100°C for 1 hour.

180 $cm^3$/h of $C_3F_6$ and 40 $cm^3$/h of $O_2$ were made to pass over 2.4 g of said catalyst (32-80 mesh) at an initial temperature of 200°C. After 3 hours at 200°C the outflowing gas had the following composition:
$O_2$ = 23.6%; $CO_2$ = 14.1%; $C_3F_6O$ = 0%; $C_3F_6$ = 62.0%

The temperature was then lowered to 150°C with the same gas flow and the following composition of the outflowing gas was observed:
$O_2$ = 26.0%; $CO_2$ = 1.15%; $C_3F_6O$ = 0.15%; $C_3F_6$ = 71.2%.

After another 3 hours under these conditions the reaction system was cooled down and the catalyst was discharged. The weight thereof was found to have increased by 17% owing to the absorbed fluorine, while the X-ray analysis revealed that most of the crystalline structure of the starting Fujasite had changed, thus giving rise to an amorphous material.

**Claims**

1. A process of oxidizing fluorinated olefins to the corresponding epoxides by directly reacting a fluoroolefin with oxygen, characterized in that the reaction is conducted in the presence of a catalyst consisting of a crystalline form of a porous silicon dioxide with an aluminium content lower than 0.1% by weight , with an X-ray diffraction pattern having the following main reflection angles: $2\vartheta = 7.94°$; $8.85°$; $23.08°$; $23.94°$, and carrying oxides of metals of the first transition series or of lanthanides or mixtures thereof.

2. The process according to claim 1, in which pure oxygen is used at a concentration, in the reaction mixture, ranging from 1 to 60% by volume.

3. The process according to claim 1, in which oxygen is fed in the form of a gaseous mixture containing at least 20% of oxygen.

4. The process according to any of the preceding claims, in which the reaction is conducted with contact times between the reactants ranging from 100 to 1000 seconds.

5. The process according to any of the preceding claims, in which the reaction is conducted at a temperature up to 250°C.

6. The process according to claim 5, in which the fluorinated olefin is tetrafluoroethylene and the reaction is conducted at a temperature below 50°C.

7. The process according to claim 5, in which the fluorinated olefin contains from 3 to 6 carbon atoms and the reaction is conducted at temperatures ranging from 50 to 250°C.

8. The process according to any of the preceding claims, in which the catalyst, prior to the oxidation reaction, is subjected to an activation step accomplished by causing to flow over said catalyst either the reactants themselves or fluoroderivatives selected from dichlorodifluoromethane, tetrafluoromethane, hexafluoroethane, tetrafluoroethylene, hexafluoropropene, gaseous hydrofluoric acid or acid fluorides such as trifluoroacetylfluoride or carbonyldifluoride, either pure or diluted with inert gases, such as nitrogen, at temperatures of from 0 to 300°C.

9. A catalyst for oxidizing fluorinated olefins to the corresponding epoxides according to the process claimed in any of the preceding claims, which catalyst comprises a carrier consisting of a crystalline form of a porous silicon dioxide with an aluminium content lower than 0.1% by weight, with an X-ray diffraction pattern having the following main reflection angles: $2\vartheta = 7.94°$; $8.85°$; $23.08°$; $23.94°$, and carrying promoters selected from oxides of metals of the first transition series of the Periodic Table or of lanthanides or from mixtures thereof.

10. The catalyst according to claim 9, in which the surface area ranges from 300 to 600 m²/g.

11. The catalyst according to claims 9 or 10, in which the promoters are carried in amounts of up to 40% by weight.

12. The catalyst according to claim 11, in which the promoters are carried in amounts of from 1 to 30% by weight.

13. The catalyst according to any of claims 9 to 13, in which the promoters are selected from copper, cobalt, cerium and chromium oxides.

**Patentansprüche**

1. Verfahren zur Oxidation fluorierter Olefine zu den entsprechenden Epoxiden durch direkte Umsetzung eines Fluorolefins mit Sauerstoff, dadurch gekennzeichnet, daß die Umsetzung in Gegenwart eines Katalysators durchgeführt wird, der aus einer kristallinen Form eines porösen Siliciumdioxids mit einem Aluminiumgehalt von weniger als 0,1 Gew.-%, mit einem Röntgenbeugungsmuster mit den folgenden Haupt-Reflexionswinkeln: $2\vartheta = 7, 94°$; $8,85°$; $23,08°$; $23,94°$; besteht, und Oxide von Metallen der ersten Übergangsreihe oder von Lanthaniden oder Mischungen davon trägt.

2. Verfahren nach Anspruch 1, worin reiner Sauerstoff bei einer Konzentration in der Reaktionsmischung im Bereich von 1 bis 60 Vol.-% verwendet wird.

3. Verfahren nach Anspruch 1, worin Sauerstoff in Form einer gasförmigen Mischung, die wenigstens 20% Sauerstoff enthält, zugeführt wird.

4. Verfahren nach irgendeinem der vorangehenden Ansprüche, worin die Umsetzung mit Kontaktzeiten zwischen den Reaktanten im Bereich von 100 bis 1000 Sekunden durchgeführt wird.

5. Verfahren nach irgendeinem der vorangehenden Ansprüche, worin die Umsetzung bei einer Temperatur von bis zu 250°C durchgeführt wird.

6. Verfahren nach Anspruch 5, worin das fluorierte Olefin Tetrafluorethylen ist und die Umsetzung bei einer Temperatur unterhalb 50°C durchgeführt wird.

7. Verfahren nach Anspruch 5, worin das fluorierte Olefin 3 bis 6 Kohlenstoffatome enthält, und die Umsetzung bei Temperaturen im Bereich von 50 bis 250°C durchgeführt wird.

8. Verfahren nach irgendeinem der vorangehenden Ansprüche, worin der Katalysator vor der Oxidationsreaktion einem Aktivierungsschritt unterworfen wird, der durchgeführt wird, indem man über den Katalysator entweder die Reaktanten selbst oder Fluorderivate, ausgewählt aus Dichlordifluormethan, Tetrafluormethan, Hexafluorethan, Tetrafluorethylen, Hexafluorpropen, gasförmiger Fluorwasserstoffsäure oder Säurefluoriden, wie z.B. Trifluoracetylfluorid oder Carbonyldifluorid, entweder unverdünnt oder mit inerten Gasen, wie z.B. Stickstoff, verdünnt, bei Temperaturen von 0 bis 300°C fließen läßt.

9. Katalysator zur Oxidation fluorierter Olefine zu den entsprechenden Epoxiden gemäß dem in irgendeinem der vorangehenden Ansprüche beanspruchten Verfahren, umfassend einen Träger, der aus einer kristallinen Form eines porösen Siliciumdioxids mit einem Aluminiumgehalt von weniger als 0,1 Gew.-%, mit einem Röntgenbeugungsmuster mit den folgenden Haupt-Reflexionswinkeln: $2\vartheta =$ 7,94°; 8,85°; 23,08°; 23,94°; besteht und Promotoren, ausgewählt aus Oxiden von Metallen der ersten Übergangsreihe des Periodensystems oder von Lanthaniden oder Mischungen davon, trägt.

10. Katalysator nach Anspruch 9, worin die Oberfläche im Bereich von 300 bis 600 m²/g liegt.

11. Katalysator nach Anspruch 9 oder 10, worin die Promotoren in einer Menge bis zu 40 Gew.-% getragen werden.

12. Katalysator nach Anspruch 11, worin die Promotoren in Mengen von 1 bis 30 Gew.-% getragen werden.

13. Katalysator nach irgendeinem der Ansprüche 9 bis 13, worin die Promotoren aus Kupfer-, Kobalt-, Cer- und Chromoxiden ausgewählt sind.

## Revendications

1. Un procédé d'oxydation d'oléfines fluorées en époxydes correspondants par réaction directe d'une fluoro-oléfine avec l'oxygène, caractérisé en ce que l'on conduit la réaction en présence d'un catalyseur constitué d'une forme cristalline d'un dioxyde de silicium poreux dont la teneur en aluminium est inférieure à 0,1% en poids, dont le schéma de diffraction aux rayons-X révèle les angles de réflexion principaux suivants:

$2\vartheta = 7{,}94°; 8{,}85°; 23{,}08°; 23{,}94°$

et supportant des oxydes de métaux des premières séries de transition ou de lanthanides ou de leurs mélanges.

2. Procédé selon la revendication 1, dans lequel on utilise de l'oxygène pur à une concentration, dans le mélange réactionnel, comprise entre 1 et 60% en volume.

3. Procédé selon la revendication 1, dans lequel l'oxygène est introduit sous la forme d'un mélange gazeux contenant au moins 20% d'oxygène.

4. Le procédé selon l'une quelconque de la revendication précédente dans lequel on conduit la réaction avec des temps de contact entre les réactifs compris entre 100 et 1000 secondes.

5. Procédé selon l'une quelconque des revendications précédentes dans lequel on conduit la réaction à une température atteignant jusqu'à 250°C.

6. Procédé selon la revendication 5 dans lequel l'oléfine fluorée est le tétrafluoroéthylène et on conduit à la réaction à une température inférieure à 50°C.

7. Procédé selon la revendication 5 dans lequel l'oléfine fluorée contient de 3 à 6 atomes de carbone et on conduit la réaction à des températures comprises entre 50 et 250°C.

8. Le procédé selon l'une quelconque des revendications précédentes dans lequel le catalyseur, avant la réaction d'oxydation, est soumis à une étape d'activation dans laquelle on fait passer sur ce catalyseur, soit les réactifs eux-mêmes, soit des fluorodérivés choisis parmi les suivants: dichlorodifluorométhane, tétrafluorométhane, hexafluoroéthane, tétrafluoroéthylène, hexafluoropropène, acide fluorhydrique gazeux ou des fluorures d'acide tel que le fluorure de trifluoroacétyle ou le difluorure de carbonyle, soit pur, soit dilué dans un gaz inerte tel que l'azote à des températures comprises entre 0 et 300°C, suivant le type d'activateur.

9. Un catalyseur d'oxydation d'oléfines fluorées en époxydes correspondants, selon le procédé revendiqué dans l'une quelconque des revendications précédentes, ce catalyseur comprenant un support constitué d'une forme cristalline d'un dioxyde de silicium poreux dont la teneur en aluminium est inférieure à 0,1% en poids et dont le schéma de diffraction aux rayons-X présente les principaux angles de réaction suivants:

$2\vartheta = 7{,}94°; 8{,}85°; 23{,}08°; 23{,}94°$

et supportant des oxydes de métaux des premières séries de transition de la Classification Périodique des Elements ou de lanthanides ou de leurs mélanges.

10. Le catalyseur selon la revendication 9, dans lequel la surface spécifique est comprise entre 300 et 600 m²/g.

11. Le catalyseur selon la revendication 9 ou 10, dans lequel les promoteurs sont supportés en quantités atteignant jusqu'à 40% en poids.

12. Le catalyseur selon la revendication 11, dans lequel les promoteurs sont supportés en quantités comprises entre 1 et 30% en poids.

13. Le catalyseur selon l'une quelconque des revendications 9 à 12, dans lequel les promoteurs sont choisis parmi cuivre, cobalt, cérium et oxydes de chrome.